(19) **Europäisches Patentamt**
**European Patent Office**
**Office européen des brevets**

(11) **EP 4 125 565 B1**

(12) **EUROPEAN PATENT SPECIFICATION**

(45) Date of publication and mention
of the grant of the patent:
**01.07.2026 Bulletin 2026/27**

(51) International Patent Classification (IPC):
***A61B 5/00*** *(2006.01)* ***A61B 5/0537*** *(2021.01)*
***A61B 5/1455*** *(2006.01)* ***A61B 90/90*** *(2016.01)*

(21) Application number: **21782145.3**

(22) Date of filing: **24.03.2021**

(52) Cooperative Patent Classification (CPC):
**A61B 5/0537; A61B 5/4875; A61B 5/6843;
A61B 90/96; G16H 40/63; G16H 50/20;
G16H 50/30;** A61B 5/117; A61B 5/742;
A61B 2562/08

(86) International application number:
**PCT/US2021/023818**

(87) International publication number:
**WO 2021/202184 (07.10.2021 Gazette 2021/40)**

(54) **BIOCAPACITANCE SENSOR**

BIOKAPAZITÄTSSENSOR

CAPTEUR DE BIOCAPACITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.04.2020 US 202063004822 P**

(43) Date of publication of application:
**08.02.2023 Bulletin 2023/06**

(73) Proprietor: **Bruin Biometrics, LLC
Los Angeles, CA 90067 (US)**

(72) Inventors:
• **BURNS, Martin F.
Los Angeles, California 90024 (US)**
• **ROSS, Graham O.
Los Angeles, California 90024 (US)**
• **GIUNTOLI, David M.
Los Angeles, California 90024 (US)**

(74) Representative: **Uexküll & Stolberg
Partnerschaft von
Patent- und Rechtsanwälten mbB
Beselerstraße 4
22607 Hamburg (DE)**

(56) References cited:
EP-A1- 0 970 656 WO-A1-2013/140714
JP-A- 2003 169 787 JP-A- 2003 169 788
US-A- 5 815 416 US-A1- 2004 041 029
US-A1- 2008 048 680 US-A1- 2010 152 551
US-A1- 2010 312 233 US-A1- 2011 223 078
US-A1- 2018 045 725 US-A1- 2018 220 924
US-A1- 2018 220 924 US-A1- 2019 290 189

• ROSS GRAHAM ET AL: "Assessment of sub-epidermal moisture by direct measurement of tissue biocapacitance", MEDICAL ENGINEERING & PHYSICS, BUTTERWORTH-HEINEMANN, GB, vol. 73, 26 July 2019 (2019-07-26), pages 92 - 99, XP085871045, ISSN: 1350-4533, [retrieved on 20190726], DOI: 10.1016/J.MEDENGPHY.2019.07.011
• YI-SHENG CHAN ET AL: "Using wireless measuring devices and Tablet PC to improve the efficiency of vital signs data collection in hospital", 2014 IEEE INTERNATIONAL SYMPOSIUM ON BIOELECTRONICS AND BIOINFORMATICS (IEEE ISBB 2014), IEEE, 11 April 2014 (2014-04-11), pages 1 - 4, XP032597759, [retrieved on 20140523], DOI: 10.1109/ISBB.2014.6820930

**Description**

CROSS-REFERENCE TO RELATED APPLICATIONS

This application claims the benefit of priority of U.S. Provisional Application 63/004,822, filed April 3, 2020.

FIELD

The present disclosure provides apparatus and methods for the noninvasive assessment of biocapacitance of living tissue.

DESCRIPTION OF THE RELATED ART

The current approach to measuring biocapacitance requires manual control of the applied pressure of the sensing device, as the measurement will vary according to the applied pressure. A means of automatically taking a measurement at the proper applied pressure without the user having to actively control the pressure will improve the repeatability of the measurement. JP 2003 169788 A (MATSUSHITA ELECTRIC IND CO LTD) 17 June 2003 (2003-06-17) describes a device for measuring biocapacitance.

SUMMARY

The present invention is defined by the claims. In an aspect, the present disclosure provides for, and includes, an apparatus for measuring biocapacitance of tissue, the apparatus comprising: a sensor comprising two electrodes; a movable element coupled to the sensor; a switch disposed between the movable element and a fixed element and configured to electrically close when a gap between the movable element and the fixed element is less than or equal to a determined value; a device coupled to the sensor and configured to make a measurement of a capacitance between the two electrodes; and a processor coupled to the switch and to the device and configured to receive the measurement from the device when the switch electrically closes.

In one aspect, the electrodes are configured such that an electric field between the electrodes penetrates into the tissue when the sensor is positioned proximate to the tissue.

In one aspect, the device is configured to repeatedly measure the capacitance between the two electrodes at a pre-determined interval.

In one aspect, the sensor further comprises an insulating cover layer coupled to the electrodes, and wherein the insulating cover layer is configured to prevent conductive contact between the electrodes and the tissue when the sensor is positioned proximate to the tissue.

In one aspect, the measurement comprises a comparison of the capacitance between the electrodes, and that of a reference capacitor.

In one aspect, the comparison comprises use of a sigma-delta method of comparing the capacitance between the electrodes to that of the reference capacitor.

In one aspect, the apparatus further comprises a visual indicator coupled to the processor, wherein the processor is further configured to activate the visual indicator upon closure of the switch.

In one aspect, the movable element is configured to move along a translation axis with respect to the fixed element, and the gap is disposed on the translation axis.

In one aspect, the apparatus further comprises a spring positioned between the movable element and the fixed element and configured to provide a monotonically increasing force along the translation axis to separate the moving element and the fixed element.

In one aspect, the movable element is further configured to allow rotation about at least one of a first rotation axis that is perpendicular to the translation axis and a second rotation axis that is perpendicular to both the translation axis and the first rotation axis.

In one aspect, the processor is further configured such that after a first measurement is received upon a first closure of the switch, the switch is to be electrically opened before a second measurement may be received.

In an aspect, the present disclosure provides for, and includes, an apparatus for measuring biocapacitance of tissue, the apparatus comprising: a sensor comprising two electrodes; a device coupled to the sensor and configured to make a measurement of a capacitance between the two electrodes; a barcode scan engine configured to optically scan a machine-readable image and determine a first alphanumeric string that is encoded in the machine-readable image; a processor coupled to the device and the engine and configured to receive the measurement from the device and to receive the first alphanumeric string from the engine.

In one aspect, the processor is further configured to receive a plurality of sequential alphanumeric strings, associate each of the sequential alphanumeric strings with one of a patient, a user, an observation, an intervention, a consumable element, a durable element, a location, and a time.

In one aspect, the processor is further configured to associate the first alphanumeric string of a patient with the sequential alphanumeric strings.

In one aspect, the processor is further configured to transfer the associated alphanumeric strings to a data system.

In an aspect, the present disclosure provides for, and includes, a method of measuring biocapacitance of tissue, the method comprising positioning a sensor that comprises a first electrode and a second electrode against a patient's skin over the tissue, measuring a capacitance between the two electrodes, optically scanning a primary machine-readable image that is associated with the patient, determining a primary alphanumeric string that is encoded in the primary machine-readable image, and associating the capacitance with the primary alphanumeric string.

In one aspect, the method further comprises optically scanning one or more secondary machine-readable images associated with one of a user, an observation, an intervention, a consumable element, a durable element, a location, and a time; determining secondary alphanumeric strings that are respectively encoded in each of the one or more secondary machine-readable images; and associating the secondary alphanumeric strings with the primary alphanumeric string.

In one aspect, the method further comprises transferring the primary and secondary alphanumeric strings to a data system.

BRIEF DESCRIPTION OF THE DRAWINGS

Aspects of the disclosure are herein described, by way of example only, with reference to the accompanying drawings. With specific reference now to the drawings in detail, it is stressed that the particulars shown are by way of example and are for purposes of illustrative discussion of aspects of the disclosure. In this regard, the description and the drawings, considered alone and together, make apparent to those skilled in the art how aspects of the disclosure may be practiced.

FIG. 1A is a plan view of a toroidal sensor according to the present disclosure.

FIG. 1B is a plan view of another aspect of a sensor according to the present disclosure.

FIG. 1C is a cross-section view of the sensor of FIG. 1A according to the present disclosure.

FIG. 1D depicts an illustrative example of an electric field between the two electrodes of the sensor of FIG. 1A according to the present disclosure.

FIG. 2 depicts the classic model of a capacitor.

FIG. 3A depicts an aspect of a biocapacitance scanner according to the present disclosure.

FIGS. 3B-3C depict details of the construction of a biocapacitance scanner according to the present disclosure.

FIGS. 4A-4C depict a sequence of states of a portion of the scanner of FIG. 3 according to the present disclosure.

FIG. 5 depicts a portion of an alternate aspect of the scanner of FIG. 3 according to the present disclosure.

FIG. 6 depicts an aspect of a visual indicator according to the present disclosure.

FIG. 7A depicts another aspect of a biocapacitance scanner according to the present disclosure.

FIG. 7B depicts an exploded view of the components of a biocapacitance scanner according to the present disclosure.

FIGS. 8A-8D depict a sequence of states of an apparatus configured to execute a sigma-delta method of measuring capacitance according to the present disclosure.

FIG. 9A depicts a hardware block diagram for measuring the capacitance of a sensor according to the present disclosure.

FIG. 9B depicts a diagram of a system for measuring, storing, transferring, and accessing measurement data according to the present disclosure.

FIG. 10 depicts a workflow that comprises scanning primary and secondary barcodes according to the present disclosure.

DETAILED DESCRIPTION

The present disclosure provides apparatuses and methods for measuring the biocapacitance of tissue. In an aspect, the present disclosure provides for, and includes, an apparatus for measuring biocapacitance of tissue, the apparatus comprising: a sensor comprising two electrodes; a movable element coupled to the sensor; a switch disposed between the movable element and a fixed element and configured to electrically close when a gap between the movable element and the fixed element is less than or equal to a determined value; a device coupled to the sensor and configured to make a measurement of a capacitance between the two electrodes; and a processor coupled to the switch and to the device and configured to receive the measurement from the device when the switch electrically closes.

In an aspect, the present disclosure provides for, and includes, an apparatus for measuring biocapacitance of tissue, the apparatus comprising: a sensor comprising two electrodes; a device coupled to the sensor and configured to make a measurement of a capacitance between the two electrodes; a barcode scan engine configured to optically scan a machine-readable image and determine a first alphanumeric string that is encoded in the machine-readable image; a processor coupled to the device and the engine and configured to receive the measurement from the device and to receive the first alphanumeric string from the engine.

In an aspect, the present disclosure provides for, and includes, a method of measuring biocapacitance of tissue, the method comprising positioning a sensor that comprises a first electrode and a second electrode against a patient's skin over the tissue, measuring a capacitance between the two electrodes, optically scanning a primary machine-readable image that is associated with the patient, determining a primary alphanumeric string that is encoded in the primary machine-readable image, and associating the capacitance with the primary alphanumeric string.

This description is not intended to be a detailed catalog of all the different ways in which the disclosure may be implemented, or all the features that may be added to the instant disclosure. For example, features illustrated with respect to one aspect may be incorporated into other aspects, and features illustrated with respect to a particular aspect may be deleted from that aspect. Thus, the disclosure contemplates that in some aspects of the disclosure, any feature or combination of features set forth herein can be excluded or omitted. In addition, numerous variations and additions to the various aspects suggested herein will be apparent to those skilled in the art in light of the instant disclosure, which do not depart from the instant disclosure. In other instances, well-known structures, interfaces, and processes have not been shown in detail in order not to unnecessarily obscure the invention. It is intended that no part of this specification be construed to effect a disavowal of any part of the full scope of the invention. Hence, the following descriptions are intended to illustrate some particular aspects of the disclosure, and not to exhaustively specify all permutations, combinations and variations thereof.

Unless otherwise defined, all technical and scientific terms used herein have the same meaning as commonly understood by one of ordinary skill in the art to which this disclosure belongs. The terminology used in the description of the disclosure herein is for the purpose of describing particular aspects or embodiments only and is not intended to be limiting of the disclosure.

All publications, patent applications, patents and other references cited herein are incorporated by reference in their entireties for the teachings relevant to the sentence and/or paragraph in which the reference is presented. References to techniques employed herein are intended to refer to the techniques as commonly understood in the art, including variations on those techniques or substitutions of equivalent techniques that would be apparent to one of skill in the art.

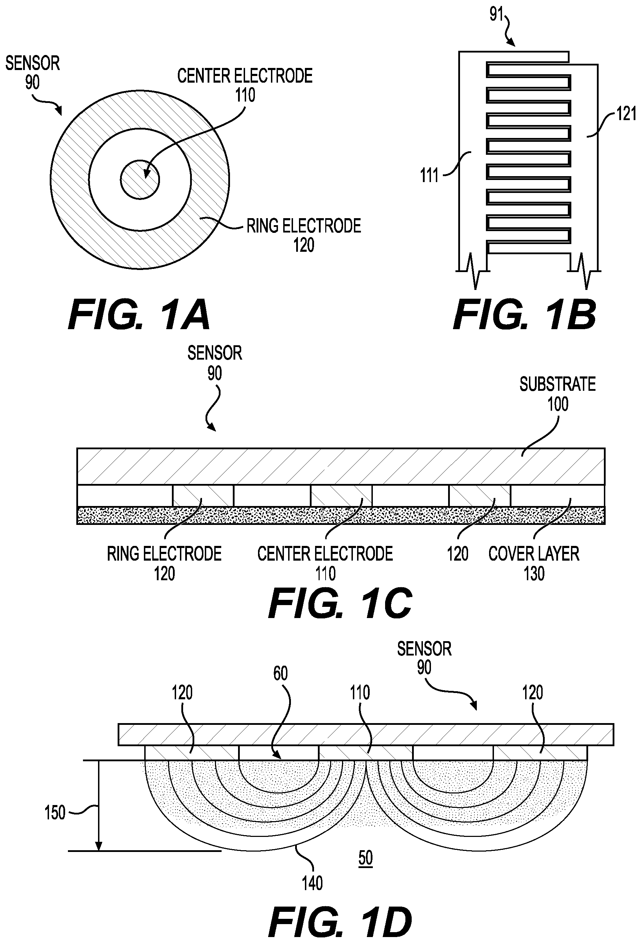

U.S. Patent Application Serial No. 14/827,375 discloses an apparatus that uses radio frequency (RF) energy to measure the sub-epidermal capacitance using a bipolar sensor similar to the sensor 90 shown in FIG. 1A. The sub-epidermal capacitance correlates with the moisture content of the target region of skin of a patient. The '375 application also discloses an array of these bipolar sensors of various sizes.

U.S. Patent Application Serial No. 15/134,110 discloses an apparatus for measuring sub-epidermal moisture (SEM) that emits and receives an RF signal at a frequency of 32 kHz through a single coaxial sensor and generates a bioimpedance signal, then converts this signal to generate an SEM value.

Both U.S. Patent Application Serial Nos. 14/827,375 and 15/134,110 are incorporated herein by reference in their entireties.

Unless the context indicates otherwise, it is specifically intended that the various features of the disclosure described herein can be used in any combination. Moreover, the present disclosure also contemplates that in some aspects of the disclosure, any feature or combination of features set forth herein can be excluded or omitted.

The methods disclosed herein include and comprise one or more steps or actions for achieving the described method. The method steps and/or actions may be interchanged with one another without departing from the scope of the present invention. In other words, unless a specific order of steps or actions is required for proper operation of the aspect, the order and/or use of specific steps and/or actions may be modified without departing from the scope of the present invention.

As used in the description of the disclosure and the appended claims, the singular forms "a," "an" and "the" are intended to include the plural forms as well, unless the context clearly indicates otherwise.

As used herein, "and/or" and "or" refer to and encompass any and all possible combinations of one or more of the associated listed items.

The terms "about" and "approximately" as used herein when referring to a measurable value such as a length, a time interval or period, a frequency, or a SEM value and the like, is meant to encompass variations of $\pm$ 20%, $\pm$ 10%, $\pm$ 5%, $\pm$ 1%, $\pm$ 0.5%, or even $\pm$ 0.1% of the specified amount.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

As used herein, the term "sub-epidermal moisture" or "SEM" refers to the moisture level that is contained in a tissue below the epidermis. An increase in tissue fluid and local edema may be caused by vascular leakiness and other changes that modify the underlying structure of the damaged tissue in the presence of continued pressure on tissue, including but not limited to apoptosis, necrosis, and the inflammatory process.

As used herein, the term "tissue biocapacitance" refers to a biophysical marker for detecting initial tissue damage based on the increased level of fluids that build up in the interstitial space.

As used herein, a "system" may be a collection of devices that are physically coupled or in wired or wireless communication with each other.

As used herein, a "patient" may be a human or an animal subject.

As used herein, "healthy" may describe tissue that does not exhibit symptoms of damage to cellular walls or blood vessels, where the presence of an increased amount of extra-cellular fluid (ECF) is an indication of such damage.

As used herein, a "switch" refers to a device that selectively provides an electrical connection between two elements or contacts. In one aspect, "closing" or deforming a portion of the switch forms the electrical connection between the two contacts thereby closing the circuit, and "opening" or reversing the switch to its original form breaks the electrical connection thereby opening the circuit. In one aspect, an applied pressure forms an electrical connection and removal of the force breaks the connection.

As used herein, "tissue" refers to a portion of the body of a living person or animal. Tissue may include one or more layers from the outermost stratum corneum, sub-epidermis, epidermis, and deeper layers of muscle, fat, and bone as well as internal structures such as veins, arteries, capillaries, lymph vessels, and nerves.

As used herein, "biocapacitance" refers to the capacitance of a sensor whose active field projects into tissue.

As used herein, "spring" refers to an element that has a force-deformation characteristic, wherein an applied force creates a deformation and/or a deformation creates a restorative force.

As used herein, "insulating" and similar terms refer to a property of an element that prevents significant electrical conduction through the element.

As used herein, a "machine-readable image" refers to a pattern that contains encoded information that may be observed by a machine and autonomously converted to information, for example, an alphanumeric string. In one aspect, the machine may project a beam of light and capture a portion of the reflected light. In one aspect, the machine may capture a 2D record of the image and process the image to extract the encoded information. In one aspect, the "machine-readable image" may be a radio-frequency sensitive device, such as a radio-frequency identification (RFID) tag, whether passive or active.

As used herein, an "alphanumeric string" refers to a sequence of characters that may include letters in upper or lower case in any language and numbers. An alphanumeric string may also have been encoded in digital form, for example a string of 0s and 1s, that is uniquely associated with the alphanumeric string.

As used herein, "optical" refers to a range of wavelengths of radiation that, in one aspect, comprises the "visible" spectrum of approximately 380 to 740 nanometers (nm). In one aspect, this range may comprise a portion of the infrared spectrum above about 740 nm. In one aspect, this range may comprise a portion of the ultraviolet spectrum below about 380 nm. In one aspect, a radio-frequency system may be substituted as an equivalent to an optical system.

As used herein, a "data system" refers to a system that comprises one or more of a data processing capability, data transmission capability, and/or data storage capability. This data system may be directly coupled to a first processor or may be coupled to a second processor that is communicatively coupled to the first processor. The storage element may utilize any of available volatile or non-volatile technologies including, but not limited to, solid-state drives (SSDs), spinning hard disk drives, and flash memory.

FIG. 1A is a plan view of an aspect of a sensor 90 according to the present disclosure. Toroidal sensor 90 comprises a first electrode 110, embodied as a circular pad, and a second electrode 120, embodied as a toroid around electrode 110. The aspect of FIG. 1A is axisymmetric and therefore insensitive to angular rotation.

FIG. 1B is a plan view of another aspect of a sensor 91 according to the present disclosure. In this aspect, the two electrodes 111 and 121 are configured with multiple fingers that are interleaved.

FIG. 1C is a cross-section view of the sensor 90 of FIG. 1A according to the present disclosure. In this aspect, the electrodes 110 and 120 are disposed on a common surface of substrate 100 and therefore coplanar with each other. In one aspect, the electrodes may be disposed on different layers or nonplanar surfaces of substrate 100. In one aspect, an insulating cover layer 130 may be disposed over the electrodes 110 and 120, as shown in FIG. 1C. The insulating cover layer 130 may prevent conductive contact between either electrode 110, 120 and a skin when sensor 90 is positioned against the skin. In one aspect, one or more of the electrodes 110 and 120 are exposed and in conductive contact with the skin when the sensor 90 is positioned against the skin. In one aspect, a plurality of electrodes (not shown in FIG. 1C) may be provided on substrate 100 and formation of a sensor 90 is controlled by selective connection of a measurement circuit (not shown in FIG. 1C) to a first electrode and a second electrode from the plurality of electrodes.

FIG. 1D depicts an illustrative example of an electric field 140 between the two electrodes 110 and 120 of sensor 90 of FIG. 1A according to the present disclosure. The electrodes 110 and 120 are positioned against the skin 60 of tissue 50 of a patient. A cover layer 130 has been omitted from Fig. 1D for clarity and could be disposed between the electrodes 110, 120 and the skin 60. The field 140 has an effective depth 150 below the skin 60.

Without being bound by any theory, the capacitance measured between electrodes 110 and 120 is partially dependent upon the dielectric constant of the tissue 50 that is within the effective field volume of the field 140. As water has a dielectric constant of approximately 81 while dry tissue has a dielectric constant of approximately 4, a small increase in the amount of water, also referred to as the sub-epidermal moisture, within the tissue may produce an increase in the capacitance measured by sensor 90.

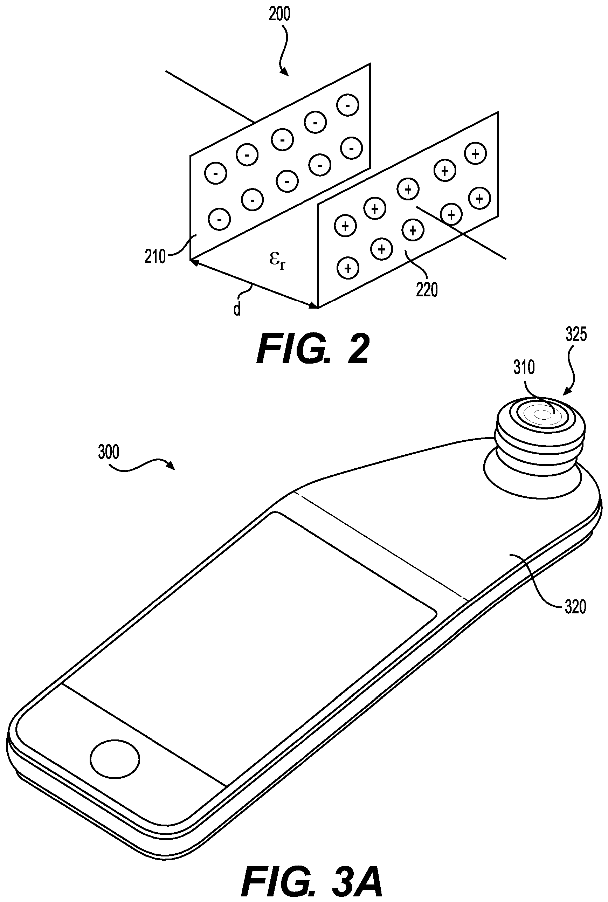

FIG. 2 depicts a classic model of a capacitor 200 according to the present disclosure. The capacitor 200 comprises a first planar electrode 210 and a second planar electrode 220 of the same dimensions that is placed parallel to electrode 210 with separation distance "d." The space between electrodes 210 and 220 is filled with a uniform material having a relative permittivity $\varepsilon_r$ and the charge on the electrodes 210 and 220 are shown as "-" and "+" signs respectively. The science of capacitors is well known to a person of ordinary skill in the art and can be found in standard electrical engineering references.

The capacitor 200 can hold an electrical charge Q. The voltage difference V between the two electrodes 210 and 220 that is created by a charge Q is dependent upon the relative permittivity $\varepsilon_r$ of the material between the electrodes. The capacitance C of the capacitor 200 can be determined by measuring the charge Q supplied to the capacitor 200 and the voltage difference V across the electrodes 210 and 220 and using the equation

$$C = \frac{Q}{V}.$$

FIG. 3A depicts an aspect of a biocapacitance scanner 300 according to the present disclosure. A sensor 310 is disposed on a "nose" 325 of the body 320.

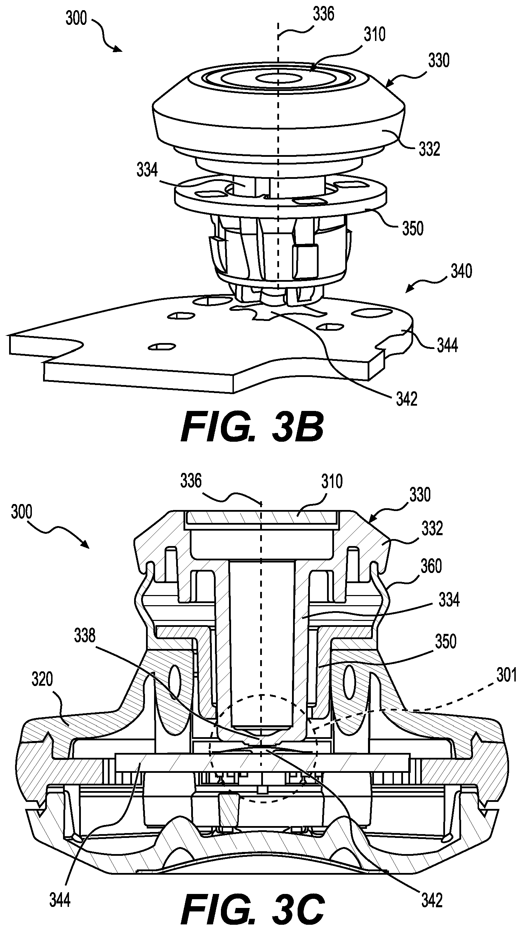

FIG. 3B depicts details of the construction of a biocapacitance scanner 300 according to the present disclosure. The sensor 300, comprising a substrate and electrodes, is fixedly coupled to a carrier 330 comprising a top 332 and a shaft 334. The shaft 334 passes through a guide 350. In one aspect, the shaft 334 can move along axis 336 relative to the guide 350. In one aspect, the top 332 can rotate relative to the shaft 334 around one or more cross-axes (not shown in Fig. 3B) that are perpendicular to axis 336. A Printed Circuit Board Assembly (PCBA) 340 comprises a substrate 344 that is disposed below the carrier 330. In one aspect, the substrate 344 is approximately perpendicular to the axis 336. In one aspect, a switch 342 is coupled to the substrate 344 and disposed directly under the shaft 334. In one aspect, the switch 342 is a dome switch that may collapse, thereby electrically closing the circuit, upon application of a force that is greater than or equal to a predetermined value, wherein the direction of the force is approximately perpendicular to the substrate 344. In one aspect, the switch 342 is configured to electrically close a circuit when the force between the sensor 310 and the skin of the patient is greater than a predetermined value. In one aspect, the switch 342 is configured to electrically close a circuit when the gap between a movable element, *e.g.* the carrier 330, and a fixed element, *e.g.* the guide 350, is less than or equal to a determined value.

In one aspect, the switch 342 is coupled to a processor (not shown in FIG. 3B) that is also coupled to a device (not shown in FIG. 3B), *e.g.* a capacitance-to-digital converter such as the AD7746 from Analog Devices, that is coupled to the sensor 310 and configured to measure the capacitance detected by the sensor 310. In one aspect, the device is configured to repeatedly measure the capacitance between the two electrodes of the sensor 310 at a pre-determined interval. In one aspect, the device measures the capacitance at a speed in the range of 1-1,000,000 times per second. In one aspect, the device measures the capacitance at a speed in the range of 1000-100,000 times per second. In one aspect, the device measures the capacitance at a speed in the range of 10,000-50,000 times per second. In one aspect, the device measures the capacitance at a speed in the range of 20,000-40,000 times per second. In one aspect, the device measures the capacitance about 34,000 times per second. In one aspect, the device measures the capacitance at this speed regardless of whether the switch 342 is electrically open or closed. In one aspect, the processor accepts a measurement from the device when the switch 342 closes. In one aspect, the processor records a plurality of measurements, *e.g.* ten sequential measurements, from the device after the switch 342 is closed. In one aspect, the processor combines the plurality of recorded measurements, e.g. by averaging, to obtain a single representative "measurement" that is used in further processing. In one aspect, the processor is reset by the opening of the switch 342, before the processor will record another measurement from the device.

FIG. 3C depicts details of the construction of a biocapacitance scanner 300 according to the present disclosure. In one aspect, the guide 350 is fixedly coupled to the body 320. The bellows 360 (not shown in FIG. 3B) is coupled at the lower edge to the guide 350 and at the upper edge to the carrier 330. In one aspect, the bellows 360 is composed of a flexible material, *e.g.* silicone, rubber, or similar, that resists compression and acts as a compression spring applying a force to separate the carrier 330 and the guide 350 along the axis 336. In one aspect, a spring is positioned between the movable element, e.g., the carrier 330, and the fixed element, e.g., the guide 350, and configured to provide a monotonically increasing force along the translation axis 336 to separate the moving element and the fixed element.

In one aspect, the shaft 334 comprises a nose 338 that is proximate to the switch 342. In one aspect, when the sensor 310 is pressed against the skin of a patient, the carrier 330 (comprising shaft 334) is configured to move toward the PCBA 344 along axis 336 until the nose 338 is in contact with switch 342 and compresses switch 342 with a pressure sufficient to close the switch 342. In one aspect, a measurement of the capacitance detected by sensor 310 occurs at the moment when the pressure applying a higher pressure on the sensor 310 does not affect the measurement of the capacitance of sensor 310, as the measurement has been taken at the time when the force first reached a level sufficient to close the switch 342.

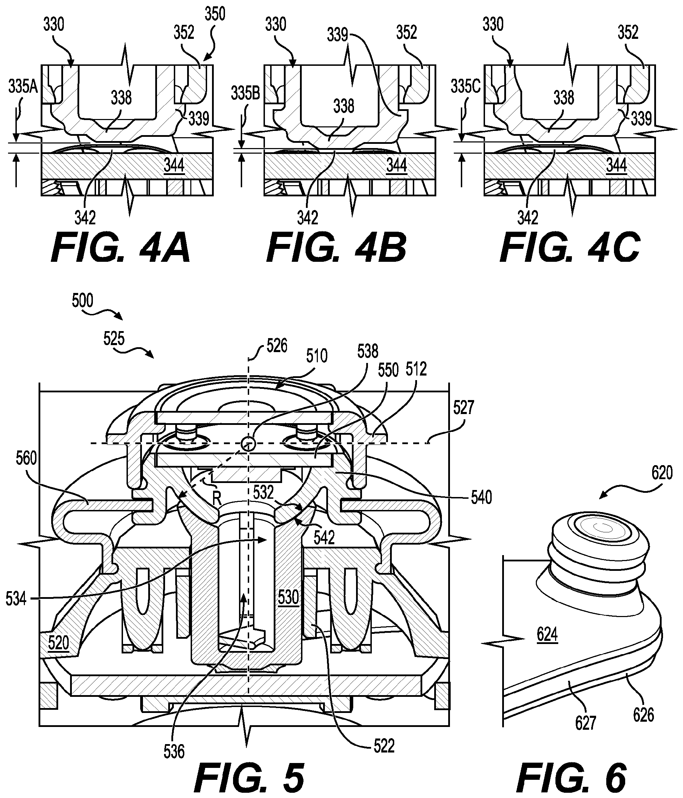

FIGS. 4A-4C depict a sequence of positional states that may be adopted by the portion of the scanner of FIG. 3C, indicated by the dashed line circle 301, according to the present disclosure.

FIG. 4A depicts a first state of a configuration of the scanner 300. In this first state, the gap 335A between nose 338 and PCBA 344 has a first value. Switch 342 protrudes from the surface of PCBA 344 in the direction of nose 338, resulting in a smaller gap between switch 342 and nose 338. Flange 339 of the carrier 330 is in contact with stop 352 of the guide 350, which is the uppermost position of the carrier 330 relative to the guide 350.

FIG. 4B depicts a second state of the same scanner 300 of FIG. 4A. A downward force has been applied to the carrier 330, thereby moving the carrier 330 downward toward the PCBA 344. The gap 335B is smaller than the gap 335A of FIG. 4A, and switch 342 has been compressed sufficient to electrically close switch 342. A further increase in the applied force may cause the carrier 330 to move further downward toward the surface of PCBA 344, but further compression of switch 342 will not affect the closure of the switch 342. In this second state, flange 339 and stop 352 are not in contact with each other.

FIG. 4C depicts a third state of the same scanner 300 of FIG. 4B after removal of some of the applied force to separate the nose 338 from the switch 342, such that switch 342 is electrically open. In one aspect, the third state is the same as the first state of FIG. 4A and flange 339 is in contact with stop 352. In one aspect, the gap 335C is smaller than gap 335A and there is a gap (not shown in FIG. 4C) between flange 339 and stop 352.

FIG. 5 depicts details of the construction of a biocapacitance scanner 500 according to the present disclosure. The scanner 500 has a head 525 with a sensor 510. In this aspect, the sensor 510 is mounted in a removable cap 512 that removably couples to the holder 540. In an aspect, the convex surface 532 of holder 540 has a radius of curvature "R" about a center 538. In one aspect, center 538 is on a surface of the interface PCBA 550. In one aspect, center 538 is on a surface of the sensor 510. In one aspect, center 538 is positioned on the axis 526.

In one aspect, carrier 530 is constrained by the guide features 522 of the body 520 to translate along axis 526. In one aspect, surface 532 of carrier 530 is concentric with surface 542 and of a radius of curvature that is slightly larger than R, thus enabling the holder 540 to rotate about center 538 while maintaining contact between portions of surfaces 532 and 542. Bellows 560 is flexible and allows rotation of holder 540 about at least one of a first rotation axis 527 that is perpendicular to translation axis 526 and a second rotation axis 528 (not visible in FIG. 5) that is perpendicular to both translation axis 526 and first rotation axis 527. As sensor 510 is fixed to cap 512, which is in turn coupled to holder 540, rotation of holder 540 also rotates the sensor 510. In one aspect, bellows 560 imparts a restorative rotational force to holder 540 to induce holder 540 to return to a centered position relative to axes 527 and 528. In one aspect, this restorative rotational force monotonically increases with an increased angle of rotation of holder 540 about one or both of axes 527 and 528.

FIG. 6 depicts an aspect of a visual indicator 627 according to the present disclosure. Body 620 comprises a front 624 and a back 626. In one aspect, a translucent gasket 627 is positioned between the front 624 and back 626. In one aspect, one or more light sources, *e.g.* one or more light emitting diodes (LEDs), are positioned proximate to an interior side of the gasket 627 such that light from the LEDs passes through the gasket 527 when the LEDs are activated and a portion of the gasket 627 appears to glow. This glowing feature is a visual indicator. In one aspect, the LEDs are coupled to a processor (not visible in FIG. 6) of the scanner, which is also coupled to a switch, *e.g.* switch 342 of FIG. 4A. In one aspect, the processor is configured to activate the LEDs, and therefore activate the visual indicator, when switch 342 closes. In one aspect, a visual indicator is provided by a portion of the body 620 that glows from internal illumination, for example from an internal LED.

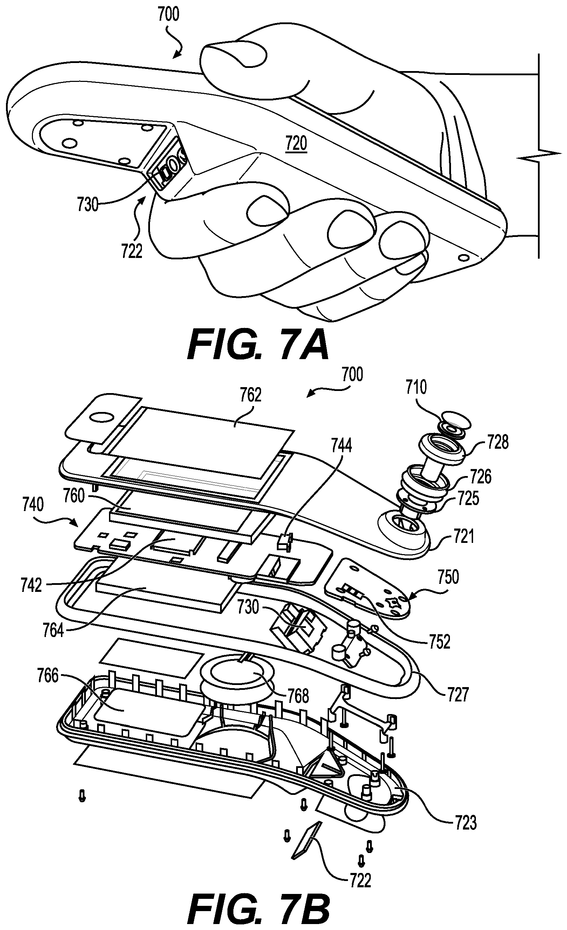

FIG. 7A depicts another aspect of a biocapacitance scanner 700 according to the present disclosure. This aspect comprises a barcode scan engine 730 mounted internal to the body 720. The scan engine 730 comprises an illuminator that emits radiation having a frequency range, for example that of visible light, and an imager that is sensitive to radiation over the frequency range emitted by the illuminator. Body 720 comprises a window 722 positioned such that a portion of the radiation projected by the illuminator passes outward through the window and the field of view of the imager includes a portion of the window. In this way, the radiation from the illuminator may illuminate an object, for example a machine-readable image printed on a patient's wristband, and the imager may obtain an image of the object, for example a barcode. In other words, the scan engine 730 optically scans a barcode, 2D matrix code, or other machine-readable encoded image. In one aspect, the scan engine comprises a signal processor that converts the image obtained by the imager into an alphanumeric string of characters. In one aspect, the scan engine 730 is coupled to a processor and provides the alphanumeric string to the processor, which is configured to receive the alphanumeric string from the scan engine. In one aspect, the alphanumeric string encodes one of a patient, a user, an observation, an intervention, a consumable element, a durable element, a location, and a time. In one aspect, the processor is further configured to receive a plurality of sequential alphanumeric strings. In one aspect, the processor is further configured to associate each of the sequential alphanumeric strings with one of a patient, a user, an observation, an intervention, a consumable element, a durable element, a location, and a time. In one aspect, the processor is further configured to associate the first alphanumeric string of a patient with the sequential alphanumeric strings. In one aspect, the processor is further configured to transfer the associated alphanumeric strings to a data system.

FIG. 7B depicts an exploded view of scanner 700 according to the present disclosure. The body 720 comprises the front 721 and the back 723, with a gasket 727 sandwiched between them when the front 721 and back 723 are brought in contact with each other. In this aspect, the front 721 is coupled to a guide 725 to which is coupled a bellows 726 and a carrier 728 to which is coupled a sensor 710. The two electrodes of the sensor 710 are coupled via electrical wires (not visible in FIG. 7B) to a device 744, *e.g.* a capacitance-to-digital converter (CDC) located, in this example, on the main board 740. The device 744 is then communicatively coupled to the processor 742. The processor 742 is also coupled via cables and wires (not visible in FIG. 7B) to display 760 which may further comprise a touchscreen. In one aspect, the processor 742 may also be coupled to one or more of the barcode scan engine 730, the battery 764, a wireless power transfer receiving coil 766, and an audible indicator 768. In this aspect, the audible indicator 768 is a piezoelectric buzzer. The display 760 is visible to a user through clear window 762 that is mounted within an opening of front 721. The processor 744 is also operatively coupled through cables to light emitting diodes (LEDs) 752 mounted, in this aspect, on a head board 750. The LEDs are positioned adjacent to the gasket 727, when scanner 700 is assembled, such that light from the LEDs 752 shines through the translucent gasket 727 to provide a visual indicator.

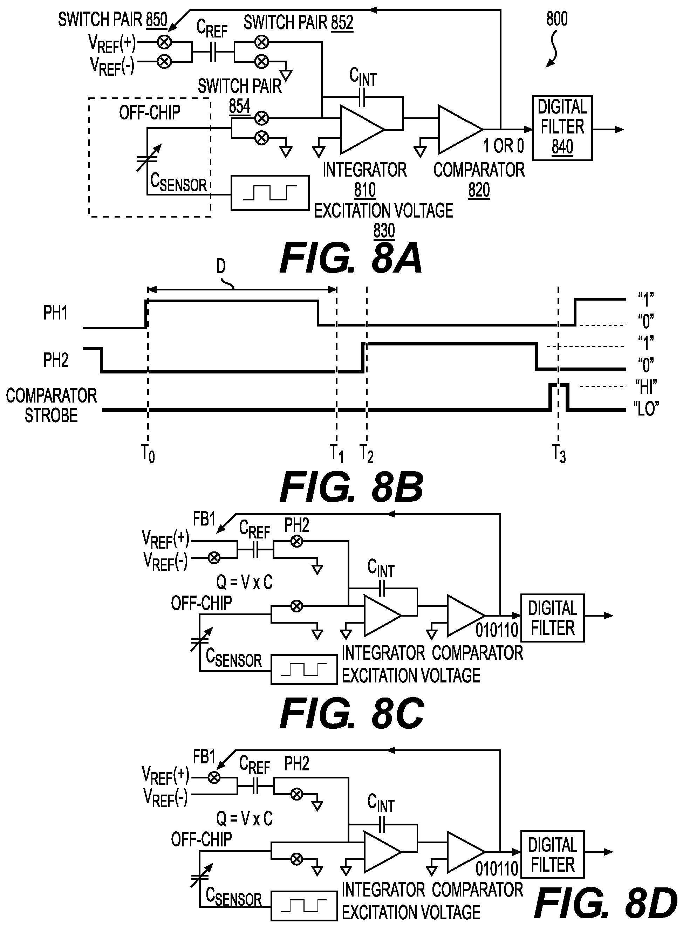

FIG. 8A depicts a schematic of circuit 800 configured to execute a sigma-delta method of measuring capacitance according to the present disclosure. The sigma delta method is well known to a person of ordinary skill in the art, and can be found in standard electrical engineering references, so only a simplified explanation is provided herein. In this diagram, the symbol ⊗ represents a controllable switch. In one aspect, circuit 800 is a part of another device, e.g. CDC 744 of Fig. 7B.

Voltage references $V_{REF}(+)$ and $V_{REF}(-)$ are selectively coupled through switch pair 850 to a reference capacitor $C_{REF}$ that is in turn selectively coupled to either ground or to the input of integrator 810. The operative configurations of these switches are described with reference to FIGS. 8C-8D. In this aspect, an off-chip capacitor $C_{SENSOR}$, for example the capacitor formed by the two electrodes of sensor 710 of the scanner 700 of Fig. 7B, is connected between a first terminal providing a square-wave excitation voltage 830 and the input of switch pair 854, which selectively couples the input to either ground or the input of integrator 810. The output of integrator 810 is coupled to an integrating capacitor $C_{INT}$ and an input of comparator 820. The output of comparator 820 will be either a "0" or a "1" signal that is fed into a digital filter 840 and controls the configurations of switch pair 850 as is described with respect to FIGS. 8C-8D.

Figure 8B illustrates the voltage states over a sample interval of the circuit of FIG. 8A, according to the present disclosure. The upper line "ph1" shows the configuration of switch pair 850, for which the "1" configuration indicates that the switch connected to $V_{REF}(+)$ is closed and the switch connected to $V_{REF}(-)$ is open while the "0" configuration indicates the reverse. The lower line "ph2" shows the configuration of switch pair 852, where the "1" configuration indicates that the switch connected to the input of integrator 810 is closed and the switch connected to ground is open, while the "0" configuration indicates the reverse.

The comparator 820 responds to the input voltage only when the "strobe" signal is "HI" and is inactive when the strobe signal is "LO." If the input is a positive voltage when the strobe is "HI" then the output of the comparator 820 is a voltage associated with a state of "1." If the input is a negative input when the strobe is "HI," the output is a voltage associated with a state of "0."

A sequence of states of circuit 800 during a single sampling cycle is as follows:

At time T0, switch pair 850 goes to "1" while switch pairs 852 and 854 are in the "0" state, as shown in FIG. 8C where solid bars across the switch symbols indicate that the switch is closed. This state is maintained for a duration "D" that is long enough for the circuit voltages to settle to a steady state. During this time, a charge Q1 has accumulated on $C_{REF}$ and a charge Q2 has accumulated on $C_{SENSOR}$. As Q = V x C, the amount of charge Q1 is determined by the voltage $V_{REF}(+)$ and the capacitance of $C_{REF}$, both of which are known and so the value of Q1 is known. Similarly, the value of Q2 is determined by the excitation voltage 830, which is known, and the capacitance $C_{SENSOR}$, which is unknown.

Around time T1, switch pair 850 reverses to state "0" while the other switch pairs 852 and 854 remain in the "0" state. This buffer interval prevents the two switches of each switch pair from simultaneously conducting.

At time T2, the switch pairs 852 and 854 change to the "1" configuration, as shown in FIG. 8D, whereupon the charges Q1 and Q2 are both provided to the input of integrator 810. This configuration effectively compares the known capacitance of the reference capacitor $C_{REF}$ to the unknown capacitance of the $C_{SENSOR}$. If the sum of Q1 and Q2 is a positive voltage, i.e. greater than the ground connected to the other input of the comparator 810, then the output of the integrator 810 will go negative. If the sum of Q1 and Q2 is a negative voltage, then the output of the integrator 810 will go positive.

At time T3, the strobe goes high and the comparator 820 responds to its input voltage and may change its output to a "1" or "0." Over a series of sampling cycles, this creates a string of 1s and 0s as an input to the digital filter 840. This is processed within the filter to determine a digital value that is equivalent to the measured capacitance of $C_{SENSOR}$. This measurement may then be provided to an external device, for example the processor 742 of Fig. 7B.

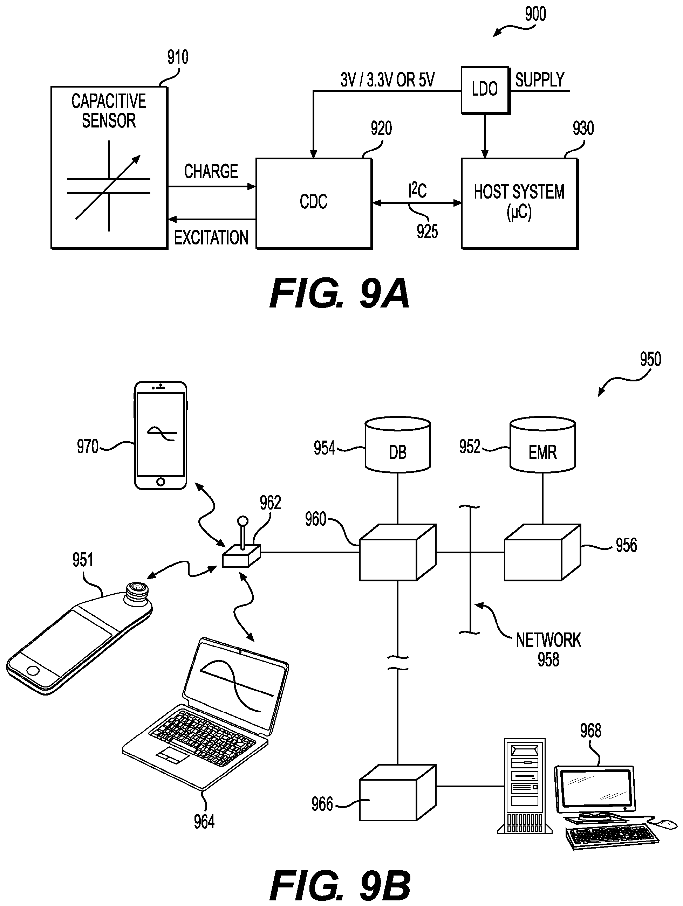

FIG. 9A depicts a hardware block diagram 900 for measuring the capacitance of a sensor 910 according to the present disclosure. The coupling of the sensor to a device 920, for example a CDC as described with reference to FIG. 7B, may consist of analog signals related to the capacitance measured at the sensor 910, as described with reference to FIGS. 8A-8D. A digital representation of the measured capacitance may be provided to a host system 930, for example processor 744 of FIG. 7B, over an inter-integrated-circuit ($I^2C$) communication line 925.

FIG. 9B depicts a schematic of an integrated system 950 for measurement, evaluation, storage, and transfer of SEM values, according to the present disclosure. In this example, system 950 comprises a scanner 951, as discussed with respect to FIG. 7B, that comprises the capability to wirelessly communicate with a WiFi access point 962. Scanner 951 may also communicate with one or more of a SEM application running on a server 960, an application running on a laptop computer 964, a smart phone 970, and other digital device. In one aspect, laptop computer 964 and smart phone 970 are held by a user of scanner 951, for example a nurse, and an application provides feedback and information to the user. In one aspect, information received from scanner 951 for a patient is stored in a database 954. In one aspect, information received from scanner 951 is transferred over a network 958 to another server 956 that stores a portion of the information in an electronic medical record (EMR) 952 of a patient. In one aspect, information from scanner 951 or retrieved from database 954 or EMR 952 is transferred to an external server 966 and then to a computer 968, for example a computer at the office of a doctor who is providing care for a patient.

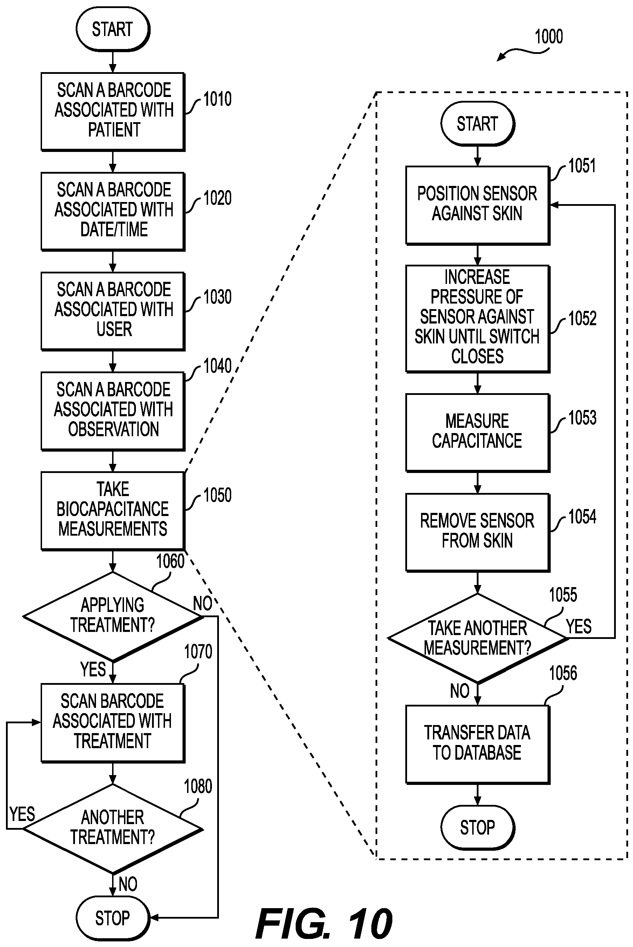

FIG. 10 depicts a workflow 1000 that comprises scanning primary and secondary barcodes according to the present disclosure. The steps shown may be performed in any order and any step may be omitted or modified.

In this example, the first steps 1010, 1020, and 1030 acquire identifying information associated with one or more of the patient, the caregiver, and the current date and time. In this example, this information is encoded in barcodes, or other machine-readable images such as a 2D matrix code, and acquired by scanning the barcode.

Step 1040 comprises acquisition of information, which may include but is not limited to observations, conditions, other measurements such a body temperature or weight, and/or other physical artifacts such as pictures or data on nutrition and hydration. In this example, this information is acquired by scanning barcodes that are associated with the various attributes, for example a set of barcodes for each element of a meal where the user scans the barcodes of items that were consumed or a series of barcodes for various amounts of liquid ingested. Step 1040 may also include scanning of barcodes associated with other aspects of care of the patient, which may include but are not limited to barcodes associated with medications being administered to a patient, barcodes associated with gowns or other general apparel, barcodes associated with equipment such as an intravenous (IV) pump being used to treat this patient as well as medical fluids or medications being administered with the IV pump, barcodes associated with treatment protocols, or any other activity or item that can be identified with a machine-readable image such as a barcode.

Step 1050 comprises activities associated with measuring sub-epidural moisture (SEM) values of the patient's body at various locations. Step 1050 comprises multiple possible steps, which are depicted as steps 1051-1056 in this example. Step 1051 comprises positioning a sensor, for example sensor 310 of scanner 300 of FIG. 3B, that comprises a first electrode and a second electrode, such as electrodes 110 and 120 of FIG. 1A, against a patient's skin over an area of tissue, for example the sacrum. Step 1052 comprises increasing the pressure of the sensor on the patient's skin until an internal switch, such as switch 342 of FIG. 4B, closes, initiates step 1053 to record the capacitance measured by the sensor. Step 1054 comprises the user removing the sensor from the skin, which resets the measurement circuit. The user then decides in step 1055 whether to take additional measurements or close the series of measurements at this location. Step 1056 transfers the SEM measurements to a database that associates the capacitance measurement with the patient identification captures in step1010. In one aspect, step 1056 may further comprise saving the data to a non-volatile local memory. In one aspect, step 1056 may not save the data at all. In one aspect, step 1056 may further comprise saving other information acquired in one or more of steps 1010-1050 to the database or local memory.

Step 1060 comprises a branching of activity depending on whether treatments will be implemented for this patient. These treatments may include but are not limited to application of bandages, ointments, or other consumables as well as use of durable products such as foot orthotics or special mattresses. These treatments may also include but are not limited to procedural treatments, for example a repositioning of the patient at a 2-hour interval compared to a standard interval of 8-hours. The treatments implemented may be related to the tissue injury being evaluated by the scanner, but do not preclude treatments related to other types of injuries or conditions. Step 1070 identifies these treatments, for this example, by scanning a barcode associated with an initiation, change, or cessation of a treatment. Step 1080 repeats this information acquisition for all treatments.

While the invention has been described with reference to particular aspects, it will be understood by those skilled in the art that various changes may be made and equivalents may be substituted for elements thereof without departing from the scope of the invention. In addition, many modifications may be made to a particular situation or material to the teachings of the invention without departing from the scope of the invention. Therefore, it is intended that the invention not be limited to the particular aspects disclosed but that the invention will include all aspects falling within the scope of the appended claims.

## Claims

1. An apparatus for measuring biocapacitance of tissue, the apparatus comprising:

   a sensor (310) comprising two electrodes,
   a movable element (330) coupled to the sensor, wherein the movable element is configured to move along a translation axis (336) with respect to a fixed element (350), wherein the movable element is further configured to allow rotation about at least one of a first rotation axis (527) that is perpendicular to the translation axis and a second rotation axis (528) that is perpendicular to both the translation axis and the first rotation axis,

a switch (342) disposed between the movable element and the fixed element and configured to electrically close when a gap (335) between the movable element and the fixed element is less than or equal to a pre-determined value,

a device coupled to the sensor and configured to make a measurement of a capacitance between the two electrodes, and

a processor coupled to the switch and to the device and configured to receive the measurement from the device when the switch electrically closes.

2. The apparatus of claim 1, wherein the electrodes are configured such that an electric field between the electrodes penetrates into the tissue when the sensor is positioned proximate to the tissue.

3. The apparatus of claim 1, wherein the device is configured to repeatedly measure the capacitance between the two electrodes at a pre-determined interval.

4. The apparatus of claim 1, wherein the sensor further comprises an insulating cover layer coupled to the electrodes, and wherein the insulating cover layer is configured to prevent conductive contact between the electrodes and the tissue when the sensor is positioned proximate to the tissue.

5. The apparatus of claim 1, wherein the measurement comprises a comparison of the capacitance between the electrodes, and that of a reference capacitor.

6. The apparatus of claim 5, wherein the comparison comprises use of a sigma-delta method of comparing the capacitance between the electrodes to that of the reference capacitor.

7. The apparatus of claim 1, further comprising:
a visual indicator coupled to the processor, wherein the processor is further configured to activate the visual indicator upon closure of the switch.

8. The apparatus of claim 1, wherein:
the gap is disposed on the translation axis.

9. The apparatus of claim 8, further comprising a spring positioned between the movable element and the fixed element and configured to provide a monotonically increasing force along the translation axis to separate the moving element and the fixed element.

10. The apparatus of claim 1, wherein the processor is further configured such that after a first measurement is received upon a first closure of the switch, the switch is to be electrically opened before a second measurement may be received.

**Patentansprüche**

1. Vorrichtung zur Messung der Biokapazität von Gewebe, umfassend:

einen Sensor (310) mit zwei Elektroden,
ein bewegliches Element (330), das mit dem Sensor gekoppelt ist, wobei das bewegliche Element konfiguriert ist, sich entlang einer Translationsachse (336) relativ zu einem feststehenden Element (350) zu bewegen, wobei das bewegliche Element weiterhin so ausgebildet ist, dass es eine Drehung um mindestens eine von einer ersten Drehachse (527), die senkrecht zur Translationsachse steht, und einer zweiten Drehachse (528), die sowohl senkrecht zur Translationsachse als auch zur ersten Drehachse steht, ermöglicht,
einen Schalter (342), der zwischen dem beweglichen Element und dem feststehenden Element angeordnet und so konfiguriert ist, dass er sich elektrisch schließt, wenn ein Spalt (335) zwischen dem beweglichen Element und dem feststehenden Element kleiner oder gleich einem vorbestimmten Wert ist,
eine Vorrichtung, die mit dem Sensor gekoppelt und so konfiguriert ist, dass sie eine Messung der Kapazität zwischen den beiden Elektroden durchführt, und
einen Prozessor, der mit dem Schalter und der Vorrichtung gekoppelt ist und so konfiguriert ist, dass er die Messung von der Vorrichtung empfängt, wenn der Schalter elektrisch schließt.

2. Vorrichtung nach Anspruch 1, wobei die Elektroden so ausgebildet sind, dass ein elektrisches Feld zwischen den Elektroden in das Gewebe eindringt, wenn der Sensor in der Nähe des Gewebes positioniert ist.

3. Vorrichtung nach Anspruch 1, wobei die Vorrichtung so konfiguriert ist, dass sie die Kapazität zwischen den beiden Elektroden in einem vorbestimmten Intervall wiederholt misst.

4. Vorrichtung nach Anspruch 1, wobei der Sensor weiterhin eine mit den Elektroden verbundene isolierende Deckschicht umfasst und, wobei die isolierende Deckschicht so ausgebildet ist, dass sie einen leitenden Kontakt zwischen den Elektroden und dem Gewebe verhindert, wenn der Sensor in der Nähe des Gewebes positioniert ist.

5. Vorrichtung nach Anspruch 1, wobei die Messung einen Vergleich der Kapazität zwischen den Elektroden und der eines Referenzkondensators umfasst.

6. Vorrichtung nach Anspruch 5, wobei der Vergleich die Verwendung eines Sigma-Delta-Verfahrens zum Vergleich der Kapazität zwischen den Elektroden mit derjenigen des Referenzkondensators umfasst.

7. Vorrichtung nach Anspruch 1, weiterhin umfassend eine mit dem Prozessor verbundene visuelle Anzeige, wobei der Prozessor ferner so konfiguriert ist, dass er die visuelle Anzeige beim Schließen des Schalters aktiviert.

8. Vorrichtung nach Anspruch 1, wobei der Spalt auf der Translationsachse angeordnet ist.

9. Vorrichtung nach Anspruch 8, die weiterhin eine Feder umfasst, die zwischen dem beweglichen Element und dem feststehenden Element angeordnet und so konfiguriert ist, dass sie eine monoton ansteigende Kraft entlang der Translationsachse bereitstellt, um das bewegliche Element und das feststehende Element voneinander zu trennen.

10. Vorrichtung nach Anspruch 1, wobei der Prozessor weiterhin so ausgelegt ist, dass nach dem Empfang einer ersten Messung bei einem ersten Schließen des Schalters der Schalter elektrisch geöffnet werden muss, bevor eine zweite Messung empfangen werden kann.

## Revendications

1. Appareil de mesure de biocapacité d'un tissu, l'appareil comprenant :

   un capteur (310) comprenant deux électrodes, un élément mobile (330) couplé au capteur, dans lequel l'élément mobile est configuré pour se déplacer le long d'un axe de translation (336) par rapport à un élément fixe (350), dans lequel l'élément mobile est en outre configuré pour permettre une rotation autour d'au moins un parmi un premier axe de rotation (527) qui est perpendiculaire à l'axe de translation et un second axe de rotation (528) qui est perpendiculaire à la fois à l'axe de translation et au premier axe de rotation, un commutateur (342) disposé entre l'élément mobile et l'élément fixe et configuré pour se fermer électriquement lorsqu'un espace (335) entre l'élément mobile et l'élément fixe est inférieur ou égal à une valeur prédéterminée,

   un dispositif couplé au capteur et configuré pour effectuer une mesure d'une capacité entre les deux électrodes, et un processeur couplé au commutateur et au dispositif et configuré pour recevoir la mesure depuis le dispositif lorsque le commutateur se ferme électriquement.

2. Appareil selon la revendication 1, dans lequel les électrodes sont configurées de telle sorte qu'un champ électrique entre les électrodes pénètre dans le tissu lorsque le capteur est positionné à proximité du tissu.

3. Appareil selon la revendication 1, dans lequel le dispositif est configuré pour mesurer de manière répétée la capacité entre les deux électrodes à un intervalle prédéterminé.

4. Appareil selon la revendication 1, dans lequel le capteur comprend en outre une couche de couverture isolante couplée aux électrodes, et dans lequel la couche de couverture isolante est configurée pour empêcher un contact conducteur entre les électrodes et le tissu lorsque le capteur est positionné à proximité du tissu.

5. Appareil selon la revendication 1, dans lequel la mesure comprend une comparaison de la capacité entre les électrodes, et celle d'un condensateur de référence.

6. Appareil selon la revendication 5, dans lequel la comparaison comprend l'utilisation d'un procédé sigma-delta de comparaison de la capacité entre les électrodes et de celle du condensateur de référence.

7. Appareil selon la revendication 1, comprenant en outre :
   un indicateur visuel couplé au processeur, dans lequel le processeur est en outre configuré pour activer l'indicateur visuel lors de la fermeture du commutateur.

8. Appareil selon la revendication 1, dans lequel :
   l'espace est disposé sur l'axe de translation.

9. Appareil selon la revendication 8, comprenant en outre un ressort positionné entre l'élément mobile et l'élément fixe et configuré pour fournir une force augmentant de manière monotone le long de l'axe de translation pour séparer l'élément mobile et l'élément fixe.

10. Appareil selon la revendication 1, dans lequel le processeur est en outre configuré de telle sorte qu'après qu'une première mesure soit reçue lors d'une première fermeture du commutateur, le commutateur doit être ouvert électriquement avant qu'une seconde mesure puisse être reçue.

SENSOR
90

CENTER ELECTRODE
110

RING ELECTRODE
120

**FIG. 1A**

91

111

121

**FIG. 1B**

SENSOR
90

SUBSTRATE
100

RING ELECTRODE
120

CENTER ELECTRODE
110

120

COVER LAYER
130

**FIG. 1C**

60

SENSOR
90

120

110

120

150

140

50

**FIG. 1D**

**FIG. 2**

**FIG. 3A**

**FIG. 3B**

**FIG. 3C**

**FIG. 4A**

**FIG. 4B**

**FIG. 4C**

**FIG. 5**

**FIG. 6**

FIG. 7A

FIG. 7B

*FIG. 8A*

*FIG. 8B*

*FIG. 8C*

*FIG. 8D*

**FIG. 9A**

**FIG. 9B**

START

SCAN A BARCODE ASSOCIATED WITH PATIENT — 1010

SCAN A BARCODE ASSOCIATED WITH DATE/TIME — 1020

SCAN A BARCODE ASSOCIATED WITH USER — 1030

SCAN A BARCODE ASSOCIATED WITH OBSERVATION — 1040

TAKE BIOCAPACITANCE MEASUREMENTS — 1050

APPLYING TREATMENT? — 1060

NO

YES

SCAN BARCODE ASSOCIATED WITH TREATMENT — 1070

ANOTHER TREATMENT? — 1080

YES

NO

STOP

1000

START

POSITION SENSOR AGAINST SKIN — 1051

INCREASE PRESSURE OF SENSOR AGAINST SKIN UNTIL SWITCH CLOSES — 1052

MEASURE CAPACITANCE — 1053

REMOVE SENSOR FROM SKIN — 1054

TAKE ANOTHER MEASUREMENT? — 1055

YES

NO

TRANSFER DATA TO DATABASE — 1056

STOP

*FIG. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63004822
- JP 2003169788 A
- US 827375
- US 134110
- US 15134110 B